(19) Europäisches Patentamt
European Patent Office
Office européen des brevets

(11) EP 1 056 787 B1

(12) # EUROPÄISCHE PATENTSCHRIFT

(45) Veröffentlichungstag und Bekanntmachung des Hinweises auf die Patenterteilung:
**06.08.2003 Patentblatt 2003/32**

(21) Anmeldenummer: **99910242.9**

(22) Anmeldetag: **19.02.1999**

(51) Int Cl.[7]: **C08F 8/30**, A61L 15/00

(86) Internationale Anmeldenummer:
**PCT/EP99/01087**

(87) Internationale Veröffentlichungsnummer:
**WO 99/042494 (26.08.1999 Gazette 1999/34)**

(54) **NACHVERNETZUNG VON HYDROGELEN MIT 2-OXAZOLIDINONEN**
SECONDARY CROSS-LINKING OF HYDROGELS WITH 2-OXAZOLIDINONES
POST-RETICULATION D'HYDROGELS AVEC DES 2-OXAZOLIDINONES

(84) Benannte Vertragsstaaten:
**BE DE ES FR GB IT NL SE**

(30) Priorität: **21.02.1998 DE 19807502**

(43) Veröffentlichungstag der Anmeldung:
**06.12.2000 Patentblatt 2000/49**

(73) Patentinhaber: **BASF AKTIENGESELLSCHAFT**
**67056 Ludwigshafen (DE)**

(72) Erfinder:
- **FUNK, Rüdiger**
**D-65527 Niedernhausen (DE)**
- **FRENZ, Volker**
**D-55246 Mainz-Kostheim (DE)**
- **RIEGEL, Ulrich**
**D-60384 Frankfurt (DE)**
- **WEISMANTEL, Matthias**
**D-63637 Jossgrund-Oberndorf (DE)**
- **ENGELHARDT, Fritz**
**Chesapeake, VA 23320 (US)**
- **DANIEL, Thomas**
**Chesapeake, VA 23321 (US)**

(56) Entgegenhaltungen:
**EP-A- 0 260 011**
**WO-A-94/09043**
**WO-A-94/27648**
**FR-A- 1 455 783**
**GB-A- 642 453**
**US-A- 3 364 181**
**US-A- 3 367 942**
**US-A- 4 056 502**
**US-A- 4 123 419**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Gel- bzw. Oberflächennachvernetzung von wasserabsorbierenden Hydrogelen durch Copolymerisation mit 2-Oxazolidinonen, die so erhältlichen Polymeren und ihre Verwendung in Hygieneartikeln, Verpackungsmaterialien und Nonwovens.

Hydrophile, hochquellfähige Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wäßrigen Flüssigkeiten quellbare Naturprodukte, wie beispielsweise Guarderivate. Solche Hydrogele werden als wäßrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie z.B. Rewet in der Windel und AUL, werden hydrophile, hochquellfähige Hydrogele im allgemeinen oberflächen- oder gelnachvernetzt. Diese Nachvernetzung ist dem Fachmann an sich bekannt und erfolgt bevorzugt in wäßriger Gelphase oder als Oberflächennachvernetzung der gemahlenen und abgesiebten Polymerpartikel.

Dazu geeignete Vernetzer sind Verbindungen, die mindestens zwei Gruppen enthalten, die mit den Carboxylgruppen des hydrophilen Polymeren kovalente Bindungen bilden können. Geeignete Verbindungen sind beispielsweise Di- oder Polyglycidylverbindungen, wie Phosphonsäurediglycidylester, Alkoxysilylverbindungen, Polyaziridine, Polyamine oder Polyamidoamine, wobei die genannten Verbindungen auch in Mischungen untereinander verwendet werdenkönnen (siehe beispielsweise EP-A-0 083 022, EP-A-0 543 303 und EP-A-0 530 438). Als Vernetzer geeignete Polyamidoamine sind insbesondere in EP-A-0 349 935 beschrieben.

Ein wesentlicher Nachteil dieser Vernetzer ist deren hohe Reaktivität, da diese besondere Schutzvorkehrungen im Produktionsbetrieb erforderlich macht, um unerwünschte Nebeneffekte zu vermeiden. Ebenso besitzen die vorgenannten Vernetzer hautreizende Eigenschaften, was bei der Verwendung in Hygieneartikeln problematisch erscheint.

Als Vernetzer sind auch polyfunktionelle Alkohole bekannt. Beispielsweise lehren EP-A-0 372 981, US-A-4 666 983 sowie US-A-5 385 983 die Verwendung von hydrophilen Polyalkoholen bzw. die Verwendung von Polyhydroxytensiden. Die Reaktion wird hiernach bei hohen Temperaturen von 120-250°C durchgeführt. Das Verfahren hat den Nachteil, daß die zur Vernetzung führende Veresterungsreaktion selbst bei diesen Temperaturen nur langsam abläuft.

Es bestand daher die Aufgabe, unter Verwendung relativ reaktionsträger, aber dennoch mit Carboxylgruppen reaktionsfähiger Verbindungen eine ebenso gute oder bessere Gel- bzw. Oberflächennachvernetzung zu erreichen. Diese Aufgabe ist so zu lösen, daß die Reaktionszeit möglichst kurz und die Reaktionstemperatur möglichst niedrig sind. Im Idealfall sollten dieselben Reaktionsbedingungen herrschen wie bei der Verwendung von hochreaktiven Epoxiden.

Überraschenderweise wurde nun gefunden, daß 2-Oxazolidinone als Vernetzer hervorragend zur Lösung dieser Aufgabe geeignet sind. Insbesondere kann die mittlere Reaktivität dieser Vernetzer durch Zugabe von anorganischen oder organischen sauren Katalysatoren gesteigert werden. Als Katalysatoren geeignet sind die bekannten anorganischen Mineralsäuren, deren saure Salze mit Alkalimetallen oder Ammonium, sowie deren entsprechender Anhydride. Geeignete organische Katalysatoren sind die bekannten Carbonsäuren, Sulfonsäuren sowie Aminosäuren.

Gegenstand der Erfindung ist ein Verfahren zur Gel- oder Oberflächennachvernetzung wasserabsorbierender Polymere, indem das Polymere mit einer Oberflächennachvernetzungslösung behandelt und während oder nach dem Behandeln durch Temperaturerhöhung nachvernetzt und getrocknet wird, wenn der Vernetzer eine Verbindung der Formel

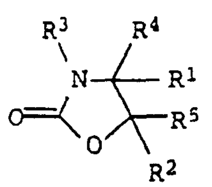

(1)

ist,
worin $R^1$ und $R^2$ unabhängig voneinander H, Hydroxy, Phenyl oder $C_1$-$C_6$-Alkyl, $R^3$ Wasserstoff, $C_1$-$C_{12}$-Alkyl, N-Hydroxy-($C_2$-$C_6$)-Alkyl, $C_1$-$C_{12}$-Alkenyl oder $C_6$-$C_{12}$-Aryl und $R^4$ und $R^5$ unabhängig voneinander $C_1$-$C_{12}$-Alkyl, $C_1$-$C_{12}$-Alkenyl, $C_6$-$C_{12}$-Aryl, Hydroxy, $C_1$-$C_{12}$-Alkoxy oder Wasserstoff bedeuten, gelöst in einem inerten Lösemittel enthält. Bevorzugt in Betracht kommende Vernetzer dieser Art sind beispielsweise 2-Oxazolidinon, N-Methyl-2-oxazolidinon

und N-Hydroxyethyl-2-oxazolidinon.

Bevorzugt zur Nachvernetzung und Trocknung ist dabei der Temperaturbereich zwischen 50 und 250°C, insbesondere 50-200°C, ganz besonders bevorzugt ist der Bereich zwischen 100-180°C. Die Aufbringung der Oberflächennachvernetzungslösung erfolgt bevorzugt durch Aufsprühen auf das Polymere in geeigneten Sprühmischern. Im Anschluß an das Aufsprühen wird das Polymerpulver thermisch getrocknet, wobei die Vernetzungsreaktion sowohl vor als auch während der Trocknung stattfinden kann. Bevorzugt ist das Aufsprühen einer Lösung des Vernetzers in Reaktionsmischern oder Misch- und Trocknungsanlagen wie beispielsweise Lödige-Mischer, BEPEX-Mischer, NAUTA-Mischer, SHUGGI-Mischer oder PROCES-SALL. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner wie z.B. ein Hordentrockner, ein Drehrohrofen, oder eine beheizbare Schnecke. Es kann aber auch z.B. eine azeotrope Destillation als Trocknungsverfahren benutzt werden. Die bevorzugte Verweilzeit bei dieser Temperatur im Reaktionsmischer oder Trockner beträgt unter 30 min, besonders bevorzugt unter 10 min.

In einer bevorzugten Ausführungsform der Erfindung wird zur Beschleunigung der Reaktion ein saurer Katalysator der Oberflächennachvernetzungslösung zugesetzt. Als Katalysator im erfindungsgemäßen Verfahren sind alle anorganischen Säuren, deren korrespondierende Anhydride bzw. organischen Säuren verwendbar. Beispiele sind Borsäure, Schwefelsäure, Iodwasserstoffsäure, Phosphorsäure, Weinsäure, Essigsäure und Toluolsulfonsäure. Insbesondere sind auch deren polymere Formen, Anhydride, sowie die sauren Salze der mehrwertigen Säuren geeignet. Beispiele hierfür sind Boroxid, Schwefeltrioxid, Diphosphorpentaoxid, und Ammoniumdihydrogenphosphat.

Der Vernetzer wird in inerten Lösemitteln gelöst. Der Vernetzer wird dabei in einer Menge von 0,01-1,0 Gew.-%, bezogen auf das eingesetzte Polymer, verwendet. Als inertes Lösemittel bevorzugt ist Wasser sowie Gemische von Wasser mit einwertigen oder mehrwertigen Alkoholen. Es können jedoch alle mit Wasser unbegrenzt mischbaren organischen Lösemittel eingesetzt werden, die nicht selbst unter den Verfahrensbedingungen reaktiv sind. Sofern ein Alkohol/Wasser-Gemisch eingesetzt wird, beträgt der Alkoholgehalt dieser Lösung beispielsweise 10-90 Gew.-%, bevorzugt 30-70 Gew.-%, insbesondere 40-60 Gew.-%. Es können alle mit Wasser unbeschränkt mischbaren Alkohole eingesetzt werden sowie Gemische mehrerer Alkohole (z.B. Methanol + Glycerin + Wasser). Die Alkoholgemische können die Alkohole in beliebigem Mischungsverhältnis enthalten. Besonders bevorzugt ist jedoch der Einsatz folgender Alkohole in wäßriger Lösung: Methanol, Ethanol, Isopropanol, Ethylenglykol und besonders bevorzugt 1,2-Propandiol sowie 1,3-Propandiol.

In einer weiteren bevorzugten Ausführungsform der Erfindung wird dieOberflächennachvernetzungslösung in einem Verhältnis von 1-20 Gew.-%, bezogen auf die Masse des Polymeren, eingesetzt. Besonders bevorzugt ist eine Lösungsmenge von 2,5-15 Gew.-%, bezogen auf das Polymer.

Ein weiterer Gegenstand der Erfindung sind vernetzte wasserabsorbierende Polymere, die nach dem erfindungsgemäßen Verfahren erhältlich sind.

Die im erfindungsgemäßen Verfahren einzusetzenden hydrophilen, hochquellfähigen Hydrogele sind insbesondere Polymere aus (co)polymerisierten hydrophilen Monomeren, Pfropf(co)polymere von einem oder mehreren hydrophilen Monomeren auf eine geeignete Pfropfgrundlage, vernetzte Cellulose- oder Stärkeether oder in wäßrigen Flüssigkeiten quellbare Naturprodukte, wie beispielsweise Guarderivate. Diese Hydrogele sind dem Fachmann bekannt und beispielsweise beschrieben in US-A-4 286 082, DE-C-27 06 135, US-A-4 340 706, DE-C-37 13 601, DE-C-28 40 010, DE-A-43 44 548, DE-A-40 20 780, DE-A-40 15 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18 172, DE-A-30 28 043, DE-A-44 18 881, EP-A-0 801 483, EP-A-0 455 985, EP-A-0 467 073, EP-A-0 312 952, EP-A-0 205 874, EP-A-0 499 774, DE-A 26 12 846, DE-A-40 20 780 EP-A-0 20 5674, US-A-5 145 906, EP-A-0 530 438, EP-A-0 670 073, US-A-4 057 521, US-A-4 062 817, US-A-4 525 527, US-A-4 295 987, US-A-5 011 892, US-A-4 076 663 oder US-A-4 931 497. Der Inhalt der vorstehend genannten Patentdokumente ist ausdrücklich Bestandteil der vorliegenden Offenbarung. Zur Herstellung dieser hydrophilen, hochquellfähigen Hydrogele geeignete hydrophile Monomere sind beispielsweise polymerisationsfähige Säuren, wie Acrylsäure, Methacrylsäure, Vinylsulfonsäure, Vinylphosphonsäure, Maleinsäure einschließlich deren Anhydrid, Fumarsäure, Itaconsäure, 2-Acrylamido-2-methylpropansulfonsäure, 2-Acrylamido-2-methylpropanphosphonsäure sowie deren Amide, Hydroxyalkylester und aminogruppen- oder ammoniumgruppenhaltige Ester und Amide sowie die Alkalimetall- und/oder Ammoniumsalze der Säuregruppen enthaltenden Monomeren. Des weiteren eignen sich wasserlösliche N-Vinylamide wie N-Vinylformamid oder Diallyldimethyl-ammoniumchlorid. Bevorzugte hydrophile Monomere sind Verbindungen der Formel

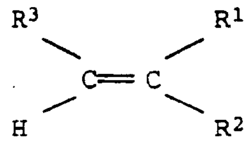 (2),

worin

$R^1$ Wasserstoff, Methyl oder Ethyl,

$R^2$ die Gruppe $-COOR^4$, eine Sulfonylgruppe oder Phosphonylgruppe, eine mit einem ($C_1$-$C_4$)-Alkanol veresterte Phosphonylgruppe oder eine Gruppe der Formel

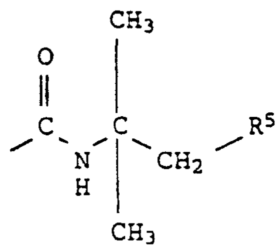 (3),

in der

$R^3$ Wasserstoff, Methyl, Ethyl oder eine Carboxylgruppe,

$R^4$ Wasserstoff, Amino-($C_1$-$C_4$)-Alkyl, Hydroxy-($C_1$-$C_4$)-Alkyl, Alkalimetall- oder Ammoniumion und

$R^5$ eine Sulfonylgruppe, eine Phosphonylgruppe, eine Carboxylgruppe oder jeweils die Alkalimetall- oder Ammoniumsalze dieser Gruppen bedeuten.

Beispiele für ($C_1$-$C_4$)-Alkanole sind Methanol, Ethanol, n-Propanol, Isopropanol oder n-Butanol.
Besonders bevorzugte hydrophile Monomere sind Acrylsäure und Methacrylsäure sowie deren Alkalimetall- oder Ammoniumsalze, z.B. Na-Acrylat, K-Acrylat oder Ammoniumacrylat.
Geeignete Pfropfgrundlagen für hydrophile Hydrogele, die durch Pfropfcopolymerisation olefinisch ungesättigter Säuren oder ihrer Alkalimetall- bzw. Ammoniumsalze erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowiehydrophile Polyester.
Geeignete Polyalkylenoxide haben beispielsweise die Formel

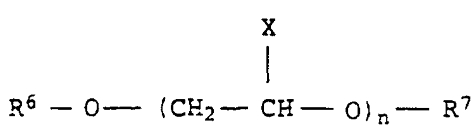 (4)

worin

$R^6$ und $R^7$ unabhängig voneinander Wasserstoff, Alkyl, Alkenyl, Phenyl oder Acyl,

X Wasserstoff oder Methyl und

n eine ganze Zahl von 1 bis 10 000 bedeuten.

$R^6$ und $R^7$ bedeuten bevorzugt Wasserstoff, ($C_1$-$C_4$)-Alkyl, ($C_2$-$C_6$)-Alkenyl oder Phenyl.

Bevorzugte Hydrogele sind insbesondere Polyacrylate, Polymethacrylate sowie die in US-A-4 931 497, US-A-5 011 892 und US-A-5 041 496 beschriebene Pfropfpolymere.

Die hydrophilen, hochquellfähigen Hydrogele sind bevorzugt vernetzt, d.h. sie enthalten Verbindungen mit mindestens zwei Doppelbindungen, die in das Polymernetzwerk einpolymerisiert sind. Geeignete Vernetzer sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, z. B. Butandiol- oder Ethylenglykoldiacrylat bzw. - methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in

EP-A-0 343 427 beschrieben sind. Besonders bevorzugt werden im erfindungsgemäßen Verfahren jedoch Hydrogele, die unter Verwendung von Polyallylethern als Vernetzer und durch saure Homopolymerisation von Acrylsäure hergestellt werden. Geeignete Vernetzer sind Pentaerythritoltri- und -tetraallylether, Polyethylenglykoldiallylether, Monoethylenglykol-diallylether, Glyceroldi- und Triallylether, Polyallylether auf Basis Sorbitol sowie ethoxylierte Varianten davon.

Das wasserabsorbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere enthalten (z.B. in Mengen von 0,001-10, vorzugsweise 0,01-1 mol-%). Ganz besonders bevorzugt sind jedoch Polymere, die über radikalische Polymerisation erhalten wurden und bei denen ein mehrfunktioneller ethylenisch ungesättigter Radikalvernetzer verwendet wurde, der zusätzlich noch mindestens eine freie Hydroxylgruppe trägt (wie. z.B. Pentaerythritoltriallylether oder Trimethylolpropandiallylether).

Die hydrophilen, hochquellfähigen Hydrogele können durch an sich bekannte Polymerisationsverfahren hergestellt werden. Bevorzugt ist die Polymerisation in wäßriger Lösung nach dem Verfahren der sogenannten Gelpolymerisation. Dabei werden z.B. 15 bis 50 gew.-%ige wäßrige Lösungen eines oder mehrerer hydrophiler Monomerer und gegebenenfalls einer geeigneten Pfropfgrundlage in Gegenwart eines Radikalinitiators, bevorzugt ohne mechanische Durchmischung, unter Ausnutzung des Trommsdorff-Norrish-Effektes (Makromol. Chem. 1, 169 (1947)), polymerisiert. Die Polymerisationsreaktion kann im Temperaturbereich zwischen 0°C und 150°C, vorzugsweise zwischen 10°C und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. Zur Auslösung der Polymerisation können energiereiche elektromagnetische Strahlen oder die üblichen chemischen Polymerisationsinitiatoren herangezogen werden, z.B. organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxiverbindungen wie $(NH_4)_2S_2O_8$, $K_2S_2O_8$ oder $H_2O_2$. Sie können gegebenenfalls in Kombination mit Reduktionsmitteln wie Natriumhydrogensulfit, und Eisen(11)-sulfat oder Redoxsystemen, welche als reduzierende Komponente eine aliphatische undaromatische Sulfinsäure, wie Benzolsulfinsäure und Toluolsulfinsäure oder Derivate dieser Säuren enthalten, wie z.B. Mannichaddukte aus Sulfinsäuren, Aldehyden und Aminoverbindungen, wie sie in der DE-C-1 301 566 beschrieben sind, verwendet werden. Durch mehrstündiges Nachheizen der Polymerisatgele im Temperaturbereich 50 bis 130°C, vorzugsweise 70 bis 100°C, können die Qualitätseigenschaften der Polymerisate noch verbessert werden.

Die erhaltenen Gele werden beispielsweise zu 0-100 mol-%, bevorzugt zu 25-100 mol-%, und besonders bevorzugt zu 50-85 mol-%, bezogen auf eingesetztes Monomer, neutralisiert, wobei die üblichen Neutralisationsmittel verwendet werden können, bevorzugt Alkalimetallhydroxide oder -oxide, besonders bevorzugt jedoch Natriumhydroxid, Natriumcarbonat und Natriumhydrogencarbonat.

Üblicherweise wird die Neutralisation durch Einmischung des Neutralisationsmittels als wäßrige Lösung oder bevorzugt auch als Feststoff erreicht. Das Gel wird hierzu mechanisch zerkleinert, z.B. mittels eines Fleischwolfes und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt. Das getrocknete Hydrogel wird hiernach gemahlen und gesiebt, wobei zur Mahlung üblicherweise Walzenstühle, Stiftmühlen oder Schwingmühlen eingesetzt werden können. Die bevorzugte Partikelgrösse des gesiebten Hydrogels liegt vorzugsweise im Bereich 45-1000 µm, besonders bevorzugt bei 45-850 µm, und ganz besonders bevorzugt bei 200-850 µm.

Zur Bestimmung der Güte der Oberflächennachvernetzung wird das getrocknete Hydrogel dann mit den Testmethoden geprüft, die nachfolgend beschrieben sind:

Methoden:

1) Zentrifugenretentionskapazität (CRC):

Bei dieser Methode wird die freie Quellbarkeit des Hydrogels im Teebeutel bestimmt. Es werden ca. 0,200 g trockenes Hydrogel in einen Teebeutel eingeschweißt (Format: 60 mm x 60 mm, Dexter 1234T-Papier) und für 30 min in eine 0,9 gew.-%ige Kochsalzlösung eingeweicht. Anschließend wird der Teebeutel 3 min in einer handelsüblichen Wäschezentrifuge (Bauknecht WS 130, 1400 U/min, Korbdurchmesser 230 mm) geschleudert. Die Bestimmung der aufgenommenen Flüssigkeitsmenge geschieht durch Auswägen des zentrifugierten Teebeutels. Zur Berücksichtigung der Aufnahmekapazität des Teebeutels selbst wird ein Blindwert bestimmt (Teebeutel ohne Hydrogel), welcher von der Auswaage (Teebeutel mit gequollenem Hydrogel) abgezogen wird.

Retention CRC [g/g] = (Auswaage Teebeutel - Blindwert - Einwaage

Hydrogel)/Einwaage Hydrogel

2) Absorption unter Druck (0,3/0,5/0,7 psi):

Bei der Absorption unter Druck werden 0.900 g trockenen Hydrogels gleichmäßig auf dem Siebboden einer Meßzelle verteilt. Die Meßzelle besteht aus einem Plexiglaszylinder (Höhe = 50 mm, Durchmesser = 60 mm), auf den als Boden ein Sieb aus Stahlgewebe (Maschenweite 36 micron bzw. 400 mesh) aufgeklebt ist. Über das gleichmäßig verteilte Hydrogel wird eine Abdeckplatte gelegt und mit einem entsprechenden Gewicht belastet. Die Zelle wird dann auf ein Filterpapier (S&S 589 Schwarzband, Durchmesser = 90 mm) gestellt, welches auf einer porösen Glasfilterplatte liegt, diese Filterplatte liegt in einer Petrischale (Höhe = 30 mm, Durchmesser = 200 mm), welche soviel 0,9 gew.-%ige Kochsalzlösung enthält, daß der Flüssigkeitsspiegel zu Beginn des Experiments identisch mit der Oberkante der Glasfritte ist. Man läßt das Hydrogel dann für 60 min die Salzlösung absorbieren.Dann nimmt man die komplette Zelle mit dem gequollenen Gel von der Filterplatte, und wägt die Apparatur nach Entfernen des Gewichts zurück.

Die Absorption unter Druck (AUL = Absorbency under load) wird wie folgt berechnet:

$$AUL\ [g/g] = ( W_b - W_a ) / W_s$$

wobei
Wb die Masse der Apparatur + Gel nach dem Quellen,
Wa die Masse der Apparatur + Einwaage vor dem Quellen,
Ws die Einwaage an trockenem Hydrogel ist.

Die Apparatur besteht aus Meßzylinder + Abdeckplatte.

Beispiel 1

In einem 40 1-Plastikeimer werden 6,9 kg reine Acrylsäure mit 23 kg Wasser verdünnt. Zu dieser Lösung fügt man 45 g Pentaerythritoltriallylether unter Rühren hinzu, und inertisiert den verschlossenen Eimer durch Durchleiten von Stickstoff. Die Polymerisation wird dann durch Zugabe von ca. 400 mg Wasserstoffperoxid und 200 mg Ascorbinsäure gestartet. Nach Beendigung der Reaktion wird das Gel mechanisch zerkleinert, und mit soviel Natronlauge versetzt bis ein Neutralisationsgrad von 75 mol-%, bezogen auf die eingesetzte Acrylsäure, erreicht wird. Das neutralisierte Gel wird dann auf einem Walzentrockner getrocknet, mit einer Stiftmühle gemahlen, und schließlich abgesiebt. Dies ist das in den nachfolgenden Beispielen verwendete Grundpolymer.

Das Grundpolymer wird in einem Waring-Labormischer mit Vernetzer-Lösung in einer solchen Menge besprüht, daß 5 % Methanol, 5 % Wasser und 0,20 % 2-Oxazolidinon, bezogen auf eingesetztes Polymer, eingesetzt werden. Anschließend wird ein Teil des feuchten Produkts bei 170°C für 60 min, und der Rest bei 170°C für 90 min im Umlufttrockenschrank getempert. Das getrocknete Produkt wird bei 850 micron abgesiebt, um Klumpen zu entfernen.

Beispiel 2

Grundpolymer gemäß Beispiel 1 wird in einem Waring-Labormischer mit Vernetzer-Lösung besprüht. Die Lösung ist dabei so zusammengesetzt, daß folgende Dosierung, bezogen auf eingesetztes Grundpolymer, erreicht

wird: 0,20 Gew.-% 2-Oxazolidinon, 5 Gew.-% Propylenglykol und 5 Gew.-% Wasser. Das feuchte Polymer wird dann bei 175°C für 40 bzw. 60 min getrocknet.

Beispiel 3

Grundpolymer gemäß Beispiel 1 wird in einem Waring-Labormischer mit Vernetzer-Lösung besprüht. Die Lösung ist dabei so zusammengesetzt, daß folgende Dosierung, bezogen auf eingesetztes Grundpolymer, erreicht wird: 0,20 Gew.-% 2-Oxazolidinon, 5 Gew.-% Propylenglykol, 5 Gew.-% Wasser und 0,2 Gew.-% Borsäure. Das feuchte Polymer wird dann bei 175°C für 30 min getrocknet.

Beispiel 4

Grundpolymer gemäß Beispiel 1 wird in einem Waring-Labormischer mit Vernetzer-Lösung besprüht. Die Lösung ist dabei so zusammengesetzt, daß folgende Dosierung, bezogen auf eingesetztes Grundpolymer, erreicht wird: 0,20 Gew.-% 2-Oxazolidinon, 5 Gew.-% Propylenglykol, 5 Gew.-% Wasser und 0,2 Gew.-% Ammoniumdihydrogenphosphat. Das feuchte Polymer wird dann bei 175°C für 30 min getrocknet.

Die gemäß obigen Beispielen hergestelltenan der Oberfläche nachvernetzten Polymere wurden wie oben beschrieben getestet. Die Ergebnisse sind in nachfolgender Tabelle zusammengefaßt:

Trocknungstemperatur und -zeit beziehen sich hierbei auf die Temperung des mit Oberflächennachvernetzungslösung besprühten Grundpolymers.

Tabelle

| Polymer | Trocknungs-temperatur | Trocknungs-zeit | Katalysator | Lösemittel | CRC [g/g] | AUL 0,3 psi [g/g] | AUL 0,7 psi-(4826,5 Pa) [g/g] |
|---|---|---|---|---|---|---|---|
| Grundpolymer (hergestellt nach Beispiel 1) | -- | -- | -- | -- | 42 | 10 | 9 |
| Nachvernetzung gemäß: | | | | | | | |
| Beispiel 1 | 170°C | 60 min | -- | Methanol/ Wasser | 35 | 36 | 21 |
| Beispiel 1 | 170°C | 90 min | -- | Methanol/ Wasser | 32 | 34 | 27 |
| Beispiel 2 | 175°C | 40 min | -- | Propylenglykol/ Wasser | 35 | 35 | 27 |
| Beispiel 2 | 175°C | 60 min | -- | Propylenglykol/ Wasser | 34 | 33 | 25 |
| Beispiel 3 | 175°C | 30 min | 0,2 % $H_3BO_3$ | Propylenglykol/ Wasser | 32 | 31 | 25 |
| Beispiel 4 | 175°C | 30 min | 0,2 % $NH_4H_2PO_4$ | Propylenglykol/ Wasser | 31 | 30 | 24 |

Beispiel 5a

Grundpolymer gemäß Beispiel 1 wird in einem ®Shuggi-Contactor mit Vernetzer-Lösung besprüht. Die Lösung ist dabei so zusammengesetzt, daß - bezogen auf eingesetztes Grundpolymer - folgende Dosierung erreicht wird: 0,20 Gew.-% 2-Oxazolidinon, 2 Gew.-% Propylenglykol und 3 Gew.-% Wasser. Das feuchte Polymer wird direkt vom Contactor in einen Scheiben-Trockner (Torus-Disc Dryer) gefördert und darin bei 185°C (Produktauslaßtemperatur) und einer Verweilzeit von 35 min. getrocknet. Das erhaltene Produkt hatte nach Absiebung des Überkorns (> 850 micron) folgende Produktdaten: CRC = 27 g/g; AUL 0,7 psi = 25 g/g.

Beispiel 5b

Völlig analog und mit den gleichen Geräten wurde mit folgender Vernetzer-Lösung das Muster 5b hergestellt: 4 Gew.-% Propylenglykol, 6 Gew.-% Wasser, 0,20 Gew.-% 2-Oxazolidinon und 0,10 Gew.-% $Al_2(SO_4)_3 \cdot 12\text{-}14\ H_2O$. Es wurde bei 175°C (Produktauslaßtemperatur) und einer Verweilzeit von 35 min getrocknet. Das erhaltene Produkt hatte nach Absiebung des Überkorns (> 850 micron) folgende Produktdaten: CRC = 31 g/g; AUL 0,7 psi = 25 g/g.

Beispiel 6

Grundpolymer gemäß Beispiel 1 wird in einem ®Shuggi-Contactor mit Vernetzer-Lösung besprüht. Die Lösung ist dabei so zusammengesetzt, daß - bezogen auf eingesetztes Grundpolymer - folgende Dosierung erreicht wird: 0,10 Gew.-% 2-Oxazolidinon, 3 Gew.-% Methanol und 7 Gew.-% Wasser. Das feuchte Polymer wird dann in einem ®Nara-Paddle-Dryer bei 185°C (Produktauslaßtemperatur) und einer Verweilzeit von 45 min getrocknet. Das erhaltene Produkt hatte nach Absiebung des Überkorns (> 850 micron) folgende Produktdaten: CRC = 26 g/g; AUL 0,7 psi = 25 g/g.

Beispiel 7a

Grundpolymer gemäß Beispiel 1 wird in einem Pflugschar-Labormischer (®ProcessA1) mit Vernetzer-Lösung besprüht. Die Lösung ist dabei so zusammengesetzt, daß folgende Dosierung, bezogen auf eingesetztes Grundpolymer, erreicht wird: 0,20 Gew.-% 2-Oxazolidinon, 4 Gew.-% 1,2-Propandiol, 6 Gew.-% Wasser sowie 0,10 Gew.-% Borsäure. Das feuchte Polymer wird dann in einem Technikumswirbelschichttrockner (®Carman Fluidized Bed Dryer) bei 200°C Wirbelschichttemperatur und einer Verweilzeit von 10 min getrocknet.

Das erhaltene Produkt hatce nach Absiebung des Überkorns (> 850 micron) folgende Produktdaten: CRC = 29 g/g; AUL 0,7 psi = 25 g/g

Beispiel 7b

Ein völlig analog hergestelltes Produkt, das bei 190°C und einer Verweilzeit von 9 min getrocknet wurde, hatte nach Absiebung des Überkorns (> 850 micron) folgende Produktdaten: CRC = 33 g/g; AUL 0,7 psi = 26 g/g.

**Patentansprüche**

1. Verfahren zur Gel- oder Oberflächennachvernetzung wasserabsorbierender Polymere, indem das Polymere mit einer Oberflächennachvernetzungslösung behandelt und während oder nach dem Behandeln durch Temperaturerhöhung nachvernetzt und getrocknet wird, **dadurch gekennzeichnet, daß** der Vernetzer eine Verbindung der Formel

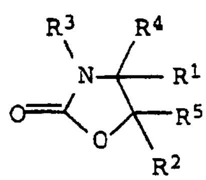

(1)

ist,
worin $R^1$ und $R^2$ unabhängig voneinander H, Hydroxy, Phenyl oder $C_1$-$C_6$-Alkyl, $R^3$ Wasserstoff, $C_1$-$C_{12}$-Alkyl,

N-Hydroxy-($C_2$-$C_6$)-Alkyl, $C_1$-$C_{12}$-Alkenyl oder $C_6$-$C_{12}$-Aryl und $R^4$ und $R^5$ unabhängig voneinander $C_1$-$C_{12}$-Alkyl, $C_1$-$C_{12}$-Alkenyl, $C_6$-$C_{12}$-Aryl, Hydroxy, $C_1$-$C_{12}$-Alkoxy oder Wasserstoff bedeuten, gelöst in einem inerten Lösemittel enthält.

**2.** Verfahren nach Anspruch 1, **dadurch gekennzeichnet, daß** das wasserabsorbierende Polymer eine polymere Acrylsäure oder ein Polyacrylat ist, insbesondere eine polymere Acrylsäure oder ein Polyacrylat, die über radikalische Polymerisation erhalten wurden und bei denen ein mehrfunktioneller ethylenisch ungesättigter Radikalvernetzer verwendet wurde, der zusätzlich noch eine oder mehrere freie Hydroxylgruppen tragen kann.

**3.** Verfahren nach Anspruch 1 und/oder 2, **dadurch gekennzeichnet, daß** zur Vernetzung ein Katalysator verwendet wird, der eine anorganische Säure, deren korrespondierendes Anhydrid, oder eine organische Säure oder deren korrespondierendes Anhydrid umfaßt.

**4.** Verfahren nach Anspruch 3, **dadurch gekennzeichnet, daß** es sich bei den Säuren um Borsäure, Schwefelsäure, Iodwasserstoffsäure, Phosphorsäure,Weinsäure, Essigsäure, Toluolsulfonsäure, sowie deren polymere Formen, Anhydride oder sauren Salze handelt.

**5.** Verfahren nach einem oder mehreren der Ansprüche 1 bis 4, **dadurch gekennzeichnet, daß** das inerte Lösemittel Wasser, ein Gemisch von Wasser mit in Wasser unbegrenzt löslichen organischen Lösemitteln oder ein Gemisch von Wasser mit einwertigen oder mehrwertigen Alkoholen ist.

**6.** Verfahren nach Anspruch 5, **dadurch gekennzeichnet, daß** bei Verwendung eines Alkohol/Wasser-Gemischs der Alkoholgehalt dieser Lösung 10-90 Gew.-%, bevorzugt 30-70 Gew.-% beträgt.

**7.** Verfahren nach Anspruch 5 und/oder 6, **dadurch gekennzeichnet, daß** der Alkohol Methanol, Ethanol, Isopropanol, Ethylenglykol, 1,2-Propandiol oder 1,3-Propandiol ist.

**8.** Verfahren nach einem oder mehreren der Ansprüche 1 bis 4, **dadurch gekennzeichnet, daß** die Oberflächennachvernetzungslösung in einem Verhältnis von 1-20 Gew.-%, insbesondere 2,5-15 Gew.-%, bezogen auf die Masse des Polymeren, eingesetzt wird.

**9.** Wasserabsorbierende Polymere, hergestellt nach dem Verfahren nach einem oder mehreren der Ansprüche 1 bis 8.

**10.** Verwendung der nach dem Verfahren nach einem oder mehreren der Ansprüche 1 bis 8 hergestellten Polymere in Hygieneartikeln, Verpackungsmaterialien und in Nonwovens.

## Claims

**1.** A process for the gel or surface postcrosslinking of water-absorbing polymers in which the polymers are treated with a surface postcrosslinking solution and during or after the treatment are postcrosslinked and dried by means of an increase in temperature, wherein the crosslinker is a compound of the formula

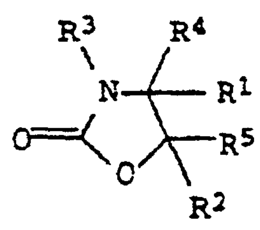

(1),

in which $R^1$ and $R^2$ independently of one another are H, hydroxyl, phenyl or $C_1$-$C_6$-alkyl, $R^3$ is hydrogen, $C_1$-$C_{12}$-alkyl, N-hydroxy($C_2$-$C_6$)-alkyl, $C_1$-$C_{12}$-alkenyl or $C_6$-$C_{12}$-aryl and $R^4$ and $R^5$ independently of one another are $C_1$-$C_{12}$-alkyl, $C_1$-$C_{12}$-alkenyl, $C_6$-$C_{12}$-aryl, hydroxyl, $C_1$-$C_{12}$-alkoxy or hydrogen, dissolved in an inert solvent.

2. The process as claimed in claim 1, wherein the water-absorbing polymer is a polymeric acrylic acid or a polyacrylate, especially a polymeric acrylic acid or polyacrylate obtained by free-radical addition polymerization using a polyfunctional ethylenically unsaturated free-radical crosslinker which may additionally carry one or more free hydroxyl groups.

3. The process as claimed in claim 1 and/or 2, wherein the catalyst used for crosslinking comprises an inorganic acid, its anhydride, or an organic acid or its anhydride.

4. The process as claimed in claim 3, wherein the acid is boric, sulfuric, hydroiodic, phosphoric, tartaric, acetic or toluenesulfonic acid or the polymeric forms, anhydrides or acid salts thereof.

5. The process as claimed in one or more of claims 1 to 4, wherein the inert solvent is water, a mixture of water with organic solvents of unlimited solubility in water, or a mixture of water with monohydric or polyhydric alcohols.

6. The process as claimed in claim 5, wherein if an alcohol/water mixture is used the alcohol content of this solution is 10-90% by weight, preferably 30-70% by weight.

7. The process as claimed in claim 5 and/or 6, wherein the alcohol is methanol, ethanol, isopropanol, ethylene glycol, 1,2-propanediol or 1,3-propanediol.

8. The process as claimed in one or more of claims 1 to 4, wherein the surface postcrosslinking solution is employed in a proportion of 1-20% by weight, in particular 2.5-15% by weight, based on the mass of the polymer.

9. A water-absorbing polymer prepared by the process as claimed in one or more of claims 1 to 8.

10. The use of a polymer prepared by the process as claimed in one or more of claims 1 to 8 in a hygiene article, packaging material or nonwoven.

## Revendications

1. Procédé de postréticulation de gel ou de surface de polymères absorbant l'eau, dans lequel le polymère est traité avec une solution de postréticulation superficielle et est postréticulé et séché pendant ou après le traitement par une élévation de température, **caractérisé en ce que** l'agent de réticulation contient un composé de la formule:

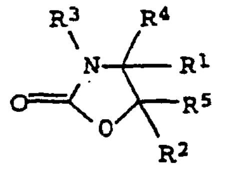

(1)

dans laquelle $R^1$ et $R^2$ représentent, indépendamment l'un de l'autre, H, de l'hydroxy, du phényle ou de l'alkyle en $C_1$-$C_6$, $R^3$ représente de l'hydrogène, de l'alkyle en $C_1$-$C_{12}$, du N-hydroxy-alkyle en $C_2$-$C_6$, de l'alcényle en $C_1$-$C_{12}$ ou de l'aryle en $C_6$-$C_{12}$ et $R^4$ et $R^5$ représentent, indépendamment l'un de l'autre, de l'alkyle en $C_1$-$C_{12}$, de l'alcényle en $C_1$-$C_{12}$, de l'aryle en $C_6$-$C_{12}$, de l'hydroxy, de l'alcoxy en $C_1$-$C_{12}$ ou de l'hydrogène, en solution dans un solvant inerte.

2. Procédé suivant la revendication 1, **caractérisé en ce que** le polymère absorbant l'eau est un acide acrylique polymère ou un polyacrylate, en particulier un acide acrylique polymère ou un polyacrylate qui a été obtenu par polymérisation radicalaire ou pour lequel on a utilisé un agent de réticulation radicalaire éthyléniquement insaturé, plurifonctionnel, qui peut en supplément porter encore un ou plusieurs groupes hydroxyle libres.

3. Procédé suivant l'une des revendications 1 et/ou 2, **caractérisé en ce que**, pour la réticulation, on utilise un catalyseur qui comporte un acide inorganique, son anhydride correspondant, ou un acide organique ou son anhydride correspondant.

**4.** Procédé suivant la revendication 3, **caractérisé en ce que**, pour ce qui concerne les acides, il s'agit d'acide borique, d'acide sulfurique, d'acide iodhydrique, d'acide phosphorique, d'acide tartrique, d'acide acétique, d'acide toluènesulfonique, ainsi que de leurs formes polymères, anhydrides ou sels acides.

**5.** Procédé suivant une ou plusieurs des revendications 1 à 4, **caractérisé en ce que** le solvant inerte est de l'eau, un mélange d'eau avec des solvants organiques solubles dans l'eau de manière illimitée ou un mélange d'eau avec des alcools à une ou plusieurs fonctions.

**6.** Procédé suivant la revendication 5, **caractérisé en ce que**, lors de l'utilisation d'un mélange d'alcool/eau, la teneur en alcool de cette solution s'élève à 10-90% en poids, de préférence à 30-70% en poids.

**7.** Procédé suivant l'une des revendications 5 et/ou 6, **caractérisé en ce que** l'alcool est du méthanol, de l'éthanol, de l'isopropanol, de l'éthylèneglycol, du 1,2-propanediol ou du 1,3-propanediol.

**8.** Procédé suivant une ou plusieurs des revendications 1 à 4, **caractérisé en ce que** la solution de postréticulation superficielle est mise en oeuvre dans des proportions de 1-20% en poids, en particulier de 2,5-15% en poids, par rapport à la masse du polymère.

**9.** Polymères absorbant l'eau, préparés selon le procédé suivant une ou plusieurs des revendications 1 à 8.

**10.** Utilisation des polymères préparés suivant le procédé selon une ou plusieurs des revendications 1 à 8, dans des articles d'hygiène, des matériaux d'emballage et des non-tissés.